# EUROPÄISCHE PATENTSCHRIFT

(11) **EP 3 277 350 B1**
(45) Veröffentlichungstag und Bekanntmachung des Hinweises auf die Patenterteilung: **08.05.2019**
(21) Anmeldenummer: 16710925.5
(22) Anmeldetag: 02.03.2016
(51) Int. Cl.: A61M 15/08, B05B 11/00

(54) **EINSATZ FÜR EINE PUMPKAPPE FÜR EINEN PHARMAZEUTISCHEN BEHÄLTER, PUMPKAPPE FÜR EINEN PHARMAZEUTISCHEN BEHÄLTER, PHARMAZEUTISCHER BEHÄLTER MIT DER PUMPKAPPE, UND COMPUTERPROGRAMMPRODUKT**
INSERT FOR A PUMP CAP FOR A PHARMACEUTICAL CONTAINER, PUMP CAP FOR A PHARMACEUTICAL CONTAINER, PHARMACEUTICAL CONTAINER WITH THE PUMP CAP, AND COMPUTER PROGRAM PRODUCT
INSERT POUR CAPUCHON DE POMPAGE POUR RÉCIPIENT À USAGE PHARMACEUTIQUE, CAPUCHON DE POMPAGE POUR RÉCIPIENT À USAGE PHARMACEUTIQUE, RÉCIPIENT À USAGE PHARMACEUTIQUE POURVU DU CAPUCHON DE POMPAGE, ET PRODUIT PROGRAMME INFORMATIQUE

(30) Priorität: 30.03.2015 DE 102015004073
(43) Veröffentlichungstag der Anmeldung: 07.02.2018
(73) Patentinhaber: Meda AB, 170 09 Solna (SE)
(72) Erfinder: TRITSCHLER, Hans-Joachim, 80639 München (DE); WEINGART, Mario, 01328 Dresden (DE); MAUS, Joachim, 63165 Mühlheim (DE)
(74) Vertreter: Schmidt, Steffen J.
(86) Internationale Anmeldenummer: PCT/EP2016/054350
(87) Internationale Veröffentlichungsnummer: WO 2016/155970

(56) Entgegenhaltungen:
- WO-A2-2014/068504
- DE-A1-102008 064 559
- KR-A- 20120 128 060
- US-A1- 2011 041 845
- US-A1- 2015 061 867
- US-B1- 8 807 131

## Beschreibung

### Technisches Gebiet, Stand der Technik, Technologischer Hintergrund

Hier wird eine Pumpkappe für einen pharmazeutischen Behälter, ein Einsatz in eine Pumpkappe für einen pharmazeutischen Behälter, ein pharmazeutischer Behälter mit der Pumpkappe, und Computerprogrammprodukt beschrieben, das zum Zusammenwirken mit einer Elektronikeinheit in der Pumpkappe dient.

Zum Beispiel zur nasalen und sublingualen Verabreichung von kontrollierten Substanzen aus Behältern sind zahlreiche aufwendige Aerosoldosierventile und Spraypumpen zum Beispiel von Aptar pharma (www.aptar.com) bekannt.

Austragvorgänge bei solchen pharmazeutischen Spendern zu erfassen, kann dazu dienen, die Austragvorgänge zu zählen. So kann der Patient oder ein Arzt sich nachfolgend einen Überblick über die ausgeführten Austragvorgänge verschaffen. Hierzu seien als technologischer Hintergrund auch die DE 10 2010 042 007 A1 und die DE 10 2008 064 559 A1 genannt.

Aus der DE 10 2014 204 939 B3 ist ein Spender in der Art eines MDI (Metered Dose Inhaler) zum Austrag eines pharmazeutischen Mediums bekannt. Dieser Spender hat einen Sensor zur Erfassung eines Austragvorgangs und eine elektronische Verarbeitungsschaltung zur Erfassung und Weiterverarbeitung eines durch den Sensor verursachten Signals. Der Sensor ist Teil einer Sensoreinheit, die einen Funksender zur Erzeugung eines Funksignals hat, und die Verarbeitungsschaltung hat einen zum Empfang des vom Funksender erzeugten Funksignals ausgebildeten Funkempfänger.

US 2015/061867 A1 bezieht sich auf Vorrichtungen und Methoden zur elektronischen Überwachung und Aufzeichnung der Abgabe von insbesondere pharmazeutischen Substanzen durch verschiedene Spendevorrichtungen, beinhaltend die drahtlose Weitergabe der aufgezeichneten Daten an eine externe elektronische Vorrichtung.

WO 2014/068504 A2 offenbart ein Inhalationsgerät zur Abgabe pharmazeutischer Substanzen, welches dazu geeignet und bestimmt ist, durch eine externe elektronische Vorrichtung die jeweils verabreichte Medikation zu dosieren.

US 8,8071,131 B1 offenbart eine Vorrichtung und eine Methode zur Überwachung des Gebrauchs eines Inhalators durch Asthmapatienten, beinhaltent ein Computerprogrammprodukt, welches zur Verwendung auf Smartphones oder persönlichen digitalen Assistenten (PDA) vorgesehen ist.

Bei diesem Spender sind somit zwei elektronische Baueinheiten vorgesehen, die in einem gemeinsamen Spender integriert, aber miteinander nicht galvanisch gekoppelt sind. Stattdessen sind die beiden elektronischen Baueinheiten, also die Sensoreinheit und die Verarbeitungsschaltung durch eine Funkschnittstelle miteinander verbunden. Diese Verarbeitungsschaltung hat eine Energiequelle (Batterie oder Akkumulator), den Funkempfänger und eine Anzeigevorrichtung an einer spezifischen Position des Spenders, sowie ggf. einen Speicher, um die Austragvorgänge unter Berücksichtigung des Zeitpunkts des Austragvorgangs abzuspeichern. Die Sensoreinheit hat den Sensor, den Funksender und ggf. eine Energiequelle (Batterie oder Akkumulator) für den Betrieb des Funksenders. Anstelle der Energiequelle ist alternativ ein Wandler zur Umwandlung mechanischer in elektrische Energie vorgesehen, die mit dem Sensor identisch ist. So wird die vom Benutzer in das System beim Austragvorgang eingebrachte Energie genutzt, um diese in elektrische Energie für den Funksender zu wandeln. Als Wandler dient ein Piezogenerator.

### Zugrundeliegendes Problem

Diese Anordnung hat eine Reihe von Nachteilen, von denen hier nur einige angesprochen seien. Es handelt sich um ein den pharmazeutischen Behälter aufnehmendes separates Gerät, das der Benutzer (Patient/in) zusätzlich zu erwerben hat. Es vergrößert das Medikamentengebinde, welche der Patient mitzuführen hat. Aber auch die Funktionsweise ist konzeptionell sehr aufwendig. Zum einen ist der Piezogenerator zur Signal- und Energie-Erzeugung teuer. Zum anderen hat die Anordnung Batterien und eine Anzeige in der im Spender ebenfalls befindlichen Verarbeitungsschaltung, die nach der Lebensphase der Anordnung zu entsorgen sind.

### Lösung des Problems

Um die vorstehend angesprochenen Probleme zumindest teilweise zu lösen, wird eine Pumpkappe für einen pharmazeutischen Behälter, ein Einsatz in eine Pumpkappe für einen pharmazeutischen Behälter ein pharmazeutischer Behälter mit der Pumpkappe, und ein Computerprogrammprodukt für einen persönlichen digitalen Assistenten vorgeschlagen.

In der Pumpkappe befindet sich eine Elektronikeinheit. Diese Elektronikeinheit ist dazu eingerichtet, einerseits eine Betätigung der Pumpkappe durch einen Benutzer zur Abgabe von Inhalt des Behälters zu erfassen, andererseits Betätigungen der Pumpkappe wiedergebende Signale drahtlos abzugeben, und sich die für das Erfassen der Betätigung und die Abgabe der Signale erforderliche Betriebs-Energie von außen drahtlos zuführen zu lassen.

Die Betriebsenergie der Elektronikeinheit wird hier durch ein von der Pumpkappe unabhängiges elektronisches Gerät in Gestalt eines sog. persönlichen digitalen Assistenten (PDA), also eines Mobil- oder Schurlostelefons, eines sog. Smartphones, einer sog. Smartwatch, eines Tablet-Computers, eines Notebooks, oder dergl. ausgesendet; die Elektronikeinheit ist dazu eingerichtet, diese Betriebsenergie von dem persönlichen digitalen Assistenten zu empfangen.

### Vorteile, Ausgestaltungen

Der pharmazeutische Behälter mit der solcherart ausgestalteten Pumpkappe ist damit wesentlich weniger aufwendig in der Herstellung und damit auch in der Entsorgung. Das elektronische Gerät hat einen von der Pumpkappe auf dem mit etwa 100 bis 200 Medikamentendosen gefüllten pharmazeutischen Behälter unabhängigen, sehr viel längeren Lebens- und Funktionszyklus als der pharmazeutische Behälter mit seiner Pumpkappe. Damit entfällt der aufwendige, zu entsorgende Teil der Spender-Anordnung aus der DE 10 2014 204 939 B3 mit Batterie und Anzeige. Hinzu kommt, dass der Benutzer in der heutigen Zeit ohnehin in der Regel einen derartigen persönlichen digitalen Assistenten (PDA) hat, den er praktisch dauernd mit sich führt. Ein Patient, der sich nasal oder sublingual kontrollierte (pharmazeutische) Substanzen zu verabreichen hat, trägt in der Regel diesen pharmazeutischen Behälter ebenfalls stets mit sich. Insofern vermeidet ein Benutzer der hier vorgeschlagenen Lösung das Mitführen überflüssiger elektronischer Geräte, da er mit der hier vorgeschlagenen Pumpkappe an dem pharmazeutischen Behälter auf die Verarbeitungsschaltung mit Batterie oder Akkumulator, den Funkempfänger und die Anzeigevorrichtung des MDI aus der DE 10 2014 204 939 B3 verzichtet. Außerdem entfällt der aufwendige, kostenintensive Piezogenerator.

Das elektronische Gerät, also der persönliche digitale Assistent, hat einerseits entsprechende strukturelle Komponenten in Gestalt einer oder mehrerer Antennen oder anderen Strahlern sowie zugehörige Ansteuerelektronik und einen Prozessor mit Speicher- und Ein-/Ausgabe-Elementen (berührungsempfindliches Anzeigepanel) vorhanden. Für den Prozessor des persönlichen digitalen Assistenten wird ein Computerprogrammprodukt (eine sog. App) vorgeschlagen, welche die Ansteuerelektronik der Antennen veranlasst, Betriebsenergie für die Elektronikeinheit der Pumpkappe abzugeben, über die Antenne von der Elektronikeinheit drahtlos abgegebene Signale zu empfangen, und nach deren Verarbeitung durch den Prozessor die Betätigung der Pumpkappe auf der Anzeige des elektronischen Geräts anzuzeigen.

Die Elektronikeinheit in der Pumpkappe ist in einer Variante ein Transmitter, zum Beispiel in Gestalt eines RFID-Moduls (RFID = Radio Frequency Identification) und hat im Wesentlichen ein Chipmodul und eine als Spule oder als Dipol ausgestaltete Antenne. Dieses Chipmodul ermöglicht ein berührungsloses, automatisiertes Einschreiben und / oder Auslesen von Daten in einen / aus einem Chip des Transmitters. Derartige Transmitter umfassen neben dem Chip eine zum Beispiel spulenförmige Transmitterantenne, die den berührungslosen Datenzugriff ermöglicht. Zu den einzuschreibenden Daten gehört eine weiter unten im Detail erläuterte Personalisierung der Pumpkappe, genauer gesagt des pharmazeutischen Behälters, zur "Paarung" mit dem Computerprogrammprodukt auf dem persönlichen digitalen Assistenten. Zu den auszulesenden Daten gehören die Signale, welche die Betätigung der Pumpkappe wiedergeben.

Der Transmitter, also die Antenne und das Chipmodul, ist in die Pumpkappe integriert und empfängt seine Betriebsenergie von dem persönlichen digitalen Assistenten, entweder nur wenn die Benutzer das entsprechende Computerprogrammprodukt auf dem persönlichen digitalen Assistenten aktiviert, oder permanent, solange die Pumpkappe auf dem pharmazeutischen Behälter sich in ausreichender räumlicher Nähe zu dem persönlichen digitalen Assistenten befindet und dieser über seine entsprechenden Antenne(n) Energie abstrahlt.

Der Transmitter ist in einer Variante dazu eingerichtet, per Nahfeld-Kommunikation (Near Field Communication = NFC) Daten per Funk auf einer kurzen Distanz von etwa 10 cm bis etwa 20 cm zu übertragen. Details hierzu sind auch in ISO / IEC 14443 A und B oder in ISO / IEC 15693 erläutert. Dabei werden die Daten für die NFC Kommunikation im 13,56 MHz Bereich übertragen. Hierzu können sowohl aktive NFC- als auch passive NFC-Transmitter eingesetzt werden. Aktive NFC Transmitter sind in der Lage Verbindungen zu initiieren und zu kommunizieren. Passive NFC Transmitter sind nicht in der Lage, eigenständig Verbindungen aufzubauen. Sie benötigen einen aktiven Partner, hier den PDA, um die Daten abzufragen.

Aktive Transmitter benötigen eine Energiequelle, während passive Transmitter ohne Energiequelle auskommen. Damit passive Transmitter ihre Informationen über den oder die Pumpvorgänge mitteilen können, machen sich diese die übertragene Energie aktiver Lesegeräte zu nutze. Durch das aktive Lesegerät wird Energie zu dem Transmitter übertragen, um die Informationen über den oder die Pumpvorgänge auf kurzer Distanz von etwa 10 cm - etwa 20 cm zu übertragen. In einer Variante ist es hier auch vorgesehen, dem Transmitter einen Betriebs-Energiespeicher, zum Bespiel in Gestalt eines Kondensators zuzuordnen.

Da eine Vielzahl von Smartphones (Apple 6, Android, Blackberry, Windows, etc.) geplant ist oder bereits existiert, die mit NFC-Funktionalität (nach dem NFC-Standard ISO / IEC 14443 A und B oder in ISO / IEC 15693), also Spulen, Ansteuerelektronik, entsprechender NFC-Ansteuersoftware (=NFC protocol stack) im Prozessor-Betriebssystem des Smartphones) ausgestattet sind, lässt sich der Einsatz / die Elektronikeinheit in der Pumpkappe mit unterschiedlichsten Smartphones oder sonstigen persönlichen digitalen Assistenten PDA mit Betriebs-Energie versorgen, ansprechen und auslesen. Ein solcher persönlicher digitaler Assistent wird für das Zusammenwirken mit der hier beschriebenen Pumpkappe bzw. dem untenstehend hier beschriebenen Einsatz (=inlay) verwendet.

Anstelle der Nahfeld-Kommunikation kann auch eine (Energie- und) Datenübertragung mittels Bluetooth-Standard, Wibree-Standard, ANT+-Standard oder Zigbee-Standard erfolgen.

Die Elektronikeinheit für die Pumpkappe ist in einer Variante mit einem Sensor ausgestattet, der einen zur Erfassung eines Austragvorgangs eingerichteten Schalter hat. Dieser Schalter kann in der besonders einfachen Form durch zwei elektrische Kontakte gebildet sein, die durch eine Metall-, z.B. Aluminiumkapsel zum Verschluss des pharmazeutischen Behälters dann elektrisch überbrückt werden, wenn der Benutzer die Pumpkappe soweit herunterdrückt, dass ein Austragvorgang erfolgt. Sollte der Verschluss des pharmazeutischen Behälters ohne Metallkapsel ausgeführt sein, ist der Schalter ein einfacher Öffner- oder Schließtaster. Anstelle dieses Schaltsensors kann auch eine kapazitive magnetische oder sonstige Erfassung des Pumpvorgangs verwendet werden, damit die Elektronikeinheit ein entsprechendes Signal abgeben kann.

In einer weiteren Variante wird ein Einsatz (inlay) in eine Pumpkappe für einen pharmazeutischen Behälter vorgeschlagen, bei dem die Elektronikeinheit für den Einsatz ein Transmitter ist. Der Transmitter hat im Wesentlichen ein Chipmodul und eine als Spule oder als Dipol ausgestaltete Antenne, zum berührungslosen, automatisierten Einschreiben und / oder Auslesen von Daten in den / aus dem Chip, wobei die einzuschreibenden Daten eine Personalisierungskennung des Einsatzes der Pumpkappe zur Paarung mit einem auf dem persönlichen digitalen Assistenten (PDA) ausgeführten Computerprogrammprodukt, und / oder die auszulesenden Daten die Signale, welche die Betätigung der Pumpkappe wiedergeben, umfassen.

Dieser Einsatz (inlay) umfasst eine Kunststoff-Folie, deren Außendurchmesser geringer ist als der Innendurchmesser der Pumpkappe. Der Einsatz ist so gestaltet, dass er in die Pumpkappe einzufügen und ggf. einzukleben ist, so dass er zwischen einem Verschluss des pharmazeutischen Behälters und einer beim Pumpvorgang sich auf den Verschluss des pharmazeutischen Behälters zubewegende Wand der Pumpkappe an letzterer ortsfest aufgenommen ist. Die Kunststoff-Folie des Einsatzes hat eine Durchtrittsöffnung für einen Medikamenten-Austragsstutzen. Die Kunststoff-Folie trägt das Chipmodul des Transmitters und die als planare Ring-Spule oder als Dipol ausgestaltete Antenne. Auch in dieser Variante ist die Elektronikeinheit mit einem Sensor ausgestattet, der einen zur Erfassung eines Austragvorgangs eingerichteten Schalter hat. Dieser Schalter kann in der besonders einfachen Form durch zwei elektrische Kontakte gebildet sein, die durch eine Metall-, z.B. Aluminiumkapsel zum Verschluss des pharmazeutischen Behälters dann elektrisch überbrückt werden, wenn der Benutzer die Pumpkappe soweit herunterdrückt, dass ein Austragvorgang erfolgt. Sollte der Verschluss des pharmazeutischen Behälters ohne Metallkapsel ausgeführt sein, ist der Schalter ein einfacher Öffner- oder Schließtaster. Im Übrigen sind aber auch kapazitive-, magnetische oder sonstige Sensor-Systeme geeignet, einen oder mehrere erfolgende Austragvorgänge zu erfassen. Durch den Transmitter werden dann die Austragvorgänge dem persönlichen digitalen Assistenten übermittelt.

Als Folienmaterial für den Einsatz kann holzfreies Papier, Beschichtungspapier oder mit Harz imprägniertes Papier, Kunststoff- bzw. Plastikfolie aus Harz verwendet werden. Beispielsweise können eines oder mehrere der folgenden Materialien wie etwa Polyethylen (PE), Polyvinylchlorid (PVC), Polyvinylchlorid-Acetat-Copolymer, Polyethylenterephthalat (PET) oder Glykol-modifiziertes Polyethylenterephthalat (PETG), Polyethylennaphthalat (PEN), Acrylnitril-Butadien-Styrol-Copolymerisat (ABS), Polyvinylbutyral (PVB), Polymethyl-methacrylat (PM-MA), Polyimid (PI), Polyvinylalkohol (PVA), Polystyrol (PS), Polyvinylphenol (PVP), Polyethylen (PE), Polypropylen (PP), Polycarbonat (PC) oder deren Derivate enthaltende Folien mit einer Dicke von mind. 75µm oder mehr verwendet werden. Mit diesem Folienmaterial ist eine ein- oder mehrlagige Trägerstruktur zu realisieren, in der eine ein- oder mehrlagige Spiral- oder zylindrische Spule als Antennenspule zur Nahfeld-Kommunikation zu realisieren ist. Außerdem kann in oder an einer solchen ein- oder mehrlagigen Trägerstruktur auch ein Kondensator zur Speicherung der über die Antennenspule empfangenen Betriebsenergie für die Elektronikeinheit aufgenommen sein.

Damit ergibt sich für den Einsatz zum Beispiel etwa eine Gestalt (Durchmesser und Höhe) einer 1-EURO-Münze mit einer zentralen Durchtrittsöffnung (einige wenige Millimeter Durchmesser oder Querabmessung) für den Medikamenten-Austragsstutzen des pharmazeutischen Behälters.

Dieser Einsatz (inlay) ist insofern besonders vorteilhaft, als er in das fertig konzipierte Gebinde aus Pumpkappe und pharmazeutischem Behälter vor der Endmontage des Gebindes in die Pumpkappe eingesetzt werden kann, ohne dass an der Pumpkappe oder dem pharmazeutischen Behälter eine Änderung vorzunehmen wäre. Durch die geringe Bauhöhe, welche durch die Dicke des Folienmaterials und des Chips sowie der Sensoranordnung gegeben ist, verändert sich auch der Betätigungsweg der Pumpkappe praktisch nicht, so dass das Austragsvolumen des Medikaments beim Betätigen der Pumpkappe mit dem in der Pumpkappe befindlichen Einsatz sich ebenfalls nicht ändert.

In einem Aspekt wird ein Gebinde bereitgestellt, mit (i) einem pharmazeutischen Behälter enthaltend ein Nasenspray, beispielsweise in Form einer Lösung oder Suspension, bevorzugt Dymista® Nasenspray (eine Azelastin HCl/Fluticasonpropionat-Kombination), (ii) eine mit einem Medikamenten-Austragsstutzen des pharmazeutischen Behälters verbundene Pumpkappe, und (iii) der Elektronikeinheit, die entweder aufgenommen ist in einem zwischen dem pharmazeutischen Behälter und der Pumpkappe angebrachten Einsatz oder direkt in der Pumpkappe. Dabei können die Pumpkappe mit der Elektronikeinheit bzw. der zwischen dem pharmazeutischen Behälter und der Pumpkappe angebrachte Einsatz in den vorstehend beschriebenen Varianten realisiert sein.

In einem weiteren Aspekt wird ein Computerprogrammprodukt für den persönlichen digitalen Assistenten PDA bereitgestellt, das dazu eingerichtet und programmiert ist, dass eine Elektronikeinheit in einem Inlay oder direkt in einer Pumpkappe nach einem der vorhergehenden Ansprüche durch entsprechende Ansteuerung des persönlichen digitalen Assistenten PDA mit Betriebsenergie versorgt wird und Austragvorgänge aus dem pharmazeutischen Behälter erfasst werden, mit einem Vorbereitungsmodus, um eine Paarung einer jeweiligen Elektronikeinheit in einem Inlay in der Pumpkappe oder direkt in einer Pumpkappe mit dem persönlichen digitalen Assistenten zu bewirken, einem Aufweckmodus, um die Elektronikeinheit in dem Inlay bzw. in der Pumpkappe zu aktivieren, einem Empfangsmodus, um darauf zu warten, dass der Benutzer die Pumpkappe betätigt, so dass die Elektronikeinheit Signale übermittelt, die Austragsvorgänge aus dem pharmazeutischen Behälter durch Betätigen der Pumpkappe wiedergeben, einem Auswertemodus, um verstrichene Zeit zwischen aufeinanderfolgenden Betätigungen der Pumpkappe zu messen und abhängig von der verstrichenen Zeit unterschiedliche Ereignisse zu kategorisieren.

Zusätzliche Details sind auch in den abhängigen Patentansprüchen definiert.

### Kurze Zeichnungsbeschreibung

Weitere Ziele, Merkmale, Vorteile und Anwendungsmöglichkeiten ergeben sich aus der nachfolgenden Beschreibung von nicht einschränkend zu verstehenden Ausführungsbeispielen mit Bezug auf die zugehörigen Zeichnungen. Dabei zeigen alle beschriebenen und / oder bildlich dargestellten Merkmale für sich oder in beliebiger Kombination den hier offenbarten Gegenstand, auch unabhängig von ihrer Gruppierung in den Ansprüchen oder deren Rückbeziehungen. Die Abmessungen und Proportionen der in den Fig. gezeigten Komponenten sind hierbei nicht unbedingt maßstäblich; sie können bei zu implementierenden Ausführungsformen vom hier Veranschaulichten abweichen.
Fig. 1 veranschaulicht eine Variante einer Pumpkappe, in einer schematischen seitlichen Schnittansicht im Zusammenwirken mit einem auf dem persönlichen digitalen Assistenten PDA ausgeführten Computerprogrammprodukt.
Fig. 1a veranschaulicht eine Variante einer Elektronikeinheit für die Pumpkappe mit einem Transmitter und einer als Spule ausgestaltete Antenne 12a in einer schematischen Darstellung.
Fig. 2 veranschaulicht eine weitere Variante einer Pumpkappe, in einer schematischen seitlichen Schnittansicht im Zusammenwirken mit einem auf dem persönlichen digitalen Assistenten ausgeführten Computerprogrammprodukt.
Fig. 2a veranschaulicht eine Variante einer Elektronikeinheit für die Pumpkappe mit einem Transmitter und einer als Spule ausgestaltete Antenne in einer schematischen Draufsicht.
Fig. 3 veranschaulicht eine weitere Variante einer Pumpkappe, in einer schematischen seitlichen Schnittansicht im Zusammenwirken mit einem auf dem persönlichen digitalen Assistenten ausgeführten Computerprogrammprodukt.

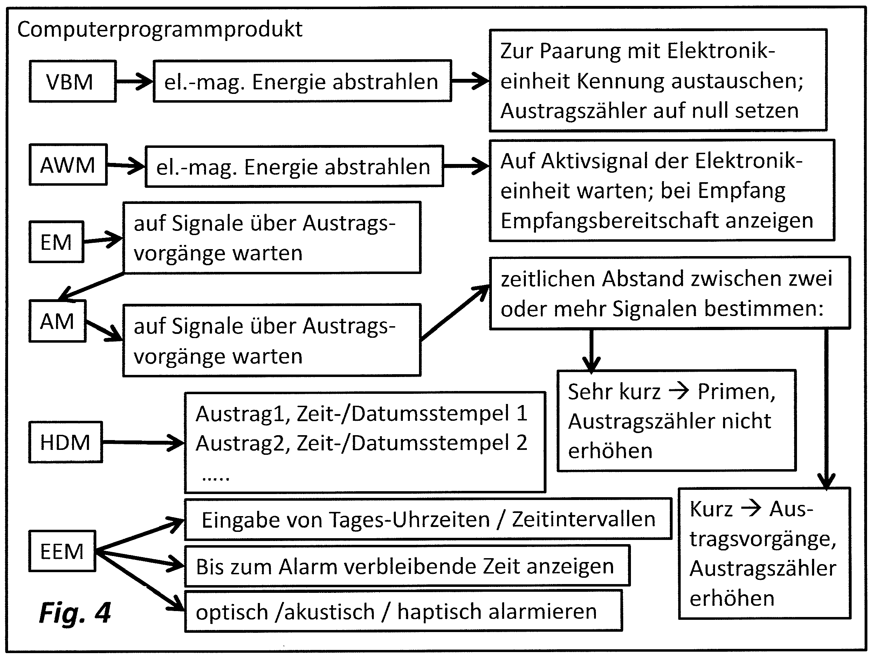

Fig. 4 veranschaulicht das Computerprogrammprodukt beim Zusammenwirken einer Elektronikeinheit in der Pumpkappe oder dem Einsatz mit dem persönlichen digitalen Assistenten, auf dem das Computerprogrammprodukt ausgeführt wird.

### Detaillierte Zeichnungsbeschreibung

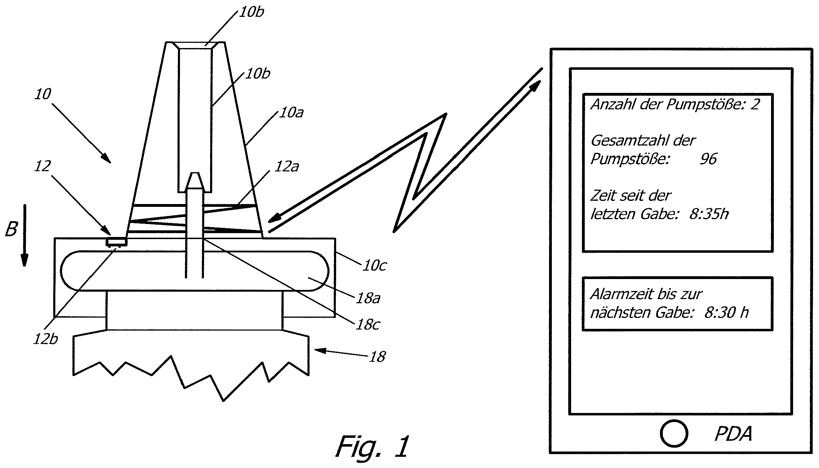

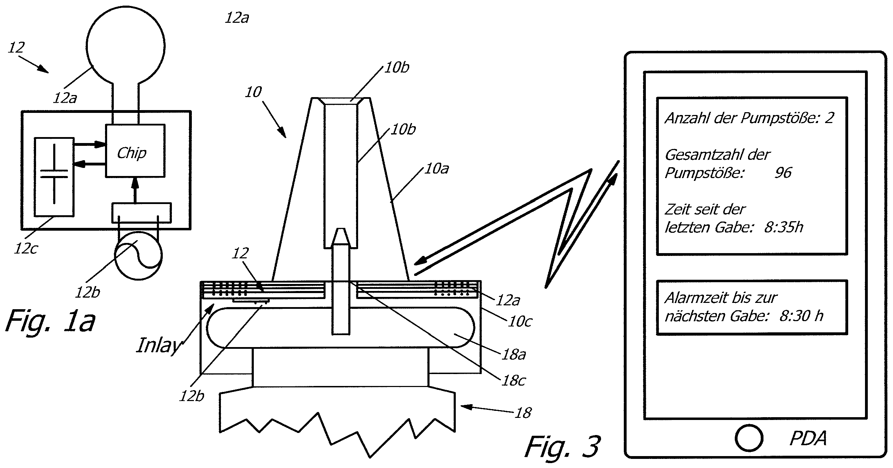

Fig. 1 zeigt eine erste Ausführungsform einer Pumpkappe 10 aus Kunststoff für einen pharmazeutischen Behälter 18. Diese Pumpkappe 10 ist als Aufsatz für den pharmazeutischen Behälter 18 ausgestaltet. Dazu hat die Pumpkappe 10 einen Applikatorstutzen 10a der sich konisch zu seinem freien Ende hin verjüngt. Der Applikatorstutzen 10a erstreckt sich von einer kreiszylindrischen Aufnahme 10c weg, die zur Aufnahme des pharmazeutischen Behälters 18 ausgebildet ist. Dieser pharmazeutische Behälter 18 hat über einen nicht weiter veranschaulichten Flüssigkeitsspeicher, eine den Hals des pharmazeutischen Behälters 18 verschließende Metall-, z.B. Aluminiumkapsel 18a und einen Auslassstutzen 18b. Die Metallkapsel 18a und der Auslassstutzen 18b sind gegeneinander verlagerbar. Dabei ist der pharmazeutische Behälter 18 derart ausgebildet, dass durch Niederdrücken der kreiszylindrischen Aufnahme 10c der Pumpkappe 10 in Richtung auf die Metallkapsel 18a der Auslassstutzen 18b in den Hals des pharmazeutischen Behälters 18 gepresst wird und dabei eine definierte Menge des zuvor im Flüssigkeitsspeicher des pharmazeutischen Behälter 18 befindlichen pharmazeutischen Mediums durch den Auslassstutzen 24 hinaus ausgetragen wird. Der Auslassstutzen 24 ist in einem zum Applikatorstutzen 10a gehörigen Austragskanal 10b aufgenommen. Die kreiszylindrische Aufnahme 10c hat eine Deckscheibe und einen rohrförmigen Abschnitt. Der rohrförmige Abschnitt ist an seinem einen (unteren) Ende offen und umgreift den Hals und ggf. teilweise den oberen Bereich des pharmazeutischen Behälters 18. Die Deckscheibe der kreiszylindrischen Aufnahme 10c schließt den rohrförmigen Abschnitt ist an seinem anderen (oberen) Ende und wird zum Ausbringen des pharmazeutischen Mediums in Richtung auf die Metallkapsel 18a des pharmazeutischen Behälters 18 gepresst.

Der Pumpkappe 10 ist eine Elektronikeinheit 12 zugeordnet. Diese ist dazu eingerichtet, eine Betätigung der Pumpkappe 10 relativ zu dem pharmazeutischen Behälter 18 durch einen Benutzer zur Abgabe von Inhalt des Behälters zu erfassen. Des Weiteren ist die Elektronikeinheit 12 dazu eingerichtet, solche Betätigungen der Pumpkappe 10 wiedergebende Signale drahtlos abzugeben. Schließlich ist die Elektronikeinheit 12 dazu eingerichtet, Betriebs-Energie zum (i) Erfassen der einen oder mehreren Betätigungen der Pumpkappe 10 durch einen Benutzer und zum (ii) drahtlosen Abgeben von die eine oder mehrere Betätigung(en) der Pumpkappe 10 wiedergebenden Signale sich von außen durch ein elektronisches Gerät, hier eines persönlichen digitalen Assistenten PDA, drahtlos zuführen zu lassen.

Wie auch in der Fig. 1a veranschaulicht, beinhaltet die Elektronikeinheit 12 einen Transmitter, der im Wesentlichen ein Chipmodul und eine als Spule oder als Dipol ausgestaltete Antenne 12a umfasst. Die Elektronikeinheit 12 ist eingerichtet zum berührungslosen, automatisierten Einschreiben und / oder Auslesen von Daten in den / aus dem Chip. Die einzuschreibenden Daten umfassen zum Beispiel eine Personalisierungskennung zur Paarung der Elektronikeinheit 12 mit einem Computerprogrammprodukt auf dem persönlichen digitalen Assistenten PDA. Damit wird sichergestellt, dass ein pharmazeutischer Behälter 18, genauer gesagt seine Pumpkappe / dessen Elektronikeinheit 12 nur dem Computerprogrammprodukt auf dem persönlichen digitalen Assistenten eines Benutzers des pharmazeutischer Behälter 18 zu zählende Pumpvorgänge kommuniziert. Die aus der Elektronikeinheit 12 auszulesenden Daten umfassen die Signale, welche die Betätigung der Pumpkappe 10 wiedergeben. Dieses auszusendende Datentelegramm kann zusätzlich die bei der Paarung von dem Computerprogrammprodukt auf dem persönlichen digitalen Assistenten PDA des jeweiligen Benutzers in die Elektronikeinheit 12 eingeschriebene Personalisierungskennung umfassen.

Über die in der Fig. 1a als Spule ausgestaltete Antenne 12a kann von der Antenne 12a aufgenommene elektromagnetische Energie, die der persönliche digitale Assistent PDA abstrahlt, in der Elektronikeinheit 12 mittels einer Energie-Gewinnungsschaltung gewonnen und als elektrische Energie in dem hier als Kondensator realisierten Betriebs-Energiespeicher 12c gespeichert werden. Somit steht die elektrische Energie zur nachfolgenden Energie-Versorgung des Chipmoduls beim Aussenden der die Betätigung der Pumpkappe 10 wiedergebenden Signale (ggf. in dem Datentelegramm mit der Personalisierungskennung) zur Verfügung.

Die Elektronikeinheit 12 des Transmitters ist in der Variante der Fig. 1 in die Pumpkappe integriert. Der Transmitter ist dazu eingerichtet, seine Betriebsenergie von dem persönlichen digitalen Assistenten entweder nur zu empfangen, wenn der Benutzer das entsprechende Computerprogrammprodukt auf dem persönlichen digitalen Assistenten PDA aktiviert. In diesem Fall strahlt der PDA die Energie über eine entsprechende Antenne ab. Alternativ dazu ist der Transmitter bzw. seine Elektronikeinheit 12 dazu eingerichtet, seine Betriebsenergie von dem persönlichen digitalen Assistenten PDA permanent zu empfangen, solange die Pumpkappe 10 auf dem pharmazeutischen Behälter 18 sich in ausreichender räumlicher Nähe zu dem persönlichen digitalen Assistenten PDA befindet und dieser über seine entsprechenden Antenne(n) Energie zu der Antrenne 12a der Elektronikeinheit 12 abstrahlt.

Die Elektronikeinheit 12 ist mit einem Sensor 12b ausgestattet, der in einer Variante einen zur Erfassung eines Austragvorgangs eingerichteten Schalter hat. Dieser Schalter kann in der besonders einfachen Form durch zwei elektrische Kontaktstellen gebildet sein, die durch eine Metallkapsel 18a zum Verschluss des pharmazeutischen Behälters 18 dann elektrisch überbrückt werden, wenn der Benutzer die Pumpkappe 10 soweit herunterdrückt, dass ein Austragvorgang erfolgt. Sollte der Verschluss des pharmazeutischen Behälters 18 ohne Metallkapsel 18a ausgeführt sein, zum Beispiel in elektrisch nicht leitendem Kunststoff ausgeführt sein, kann der Sensor 12b ein als einfacher Öffner- oder Schließtaster ausgeführter Schalter ein. Anstelle dieses Schaltsensors kann auch ein kapazitiver, magnetischer oder sonstiger Sensor 12b zur Erfassung des Pumpvorgangs vorgesehen sein, damit die Elektronikeinheit 12 ein den Austragvorgang entsprechend wiedergebendes Signal an den persönlichen digitalen Assistenten PDA abgeben kann.

Für die Elektronikeinheit 12 kommen zum Beispiel solche NFC-Komponenten der Firmen NXP (www.nxp.com) oder AMS (www.ams.com) in Frage, welche jeweils einen Signaleingang aufweisen, um Sensorsignale aufnehmen, verarbeiten, und über die Antenne 12a an den persönlichen digitalen Assistenten PDA abstrahlen zu können.

In der in Fig. 1 veranschaulichten Variante ist die Antenne 12a in das Kunststoffmaterial des Applikatorstutzens 10a der Pumpkappe 10 eingebettet. Dabei hat die als Schleife ausgebildete Spulenantenne 12a mehrere sich dem Konus des Applikatorstutzens 10a folgend verjüngende Windungen. Bei weiteren - hier nicht veranschaulichten - Varianten, kann die Spulenantenne 12a auch in der kreiszylindrischen Aufnahme 10c, zum Beispiel in der Deckscheibe der kreiszylindrischen Aufnahme 10c oder deren rohrförmigem Abschnitt aufgenommen sein.

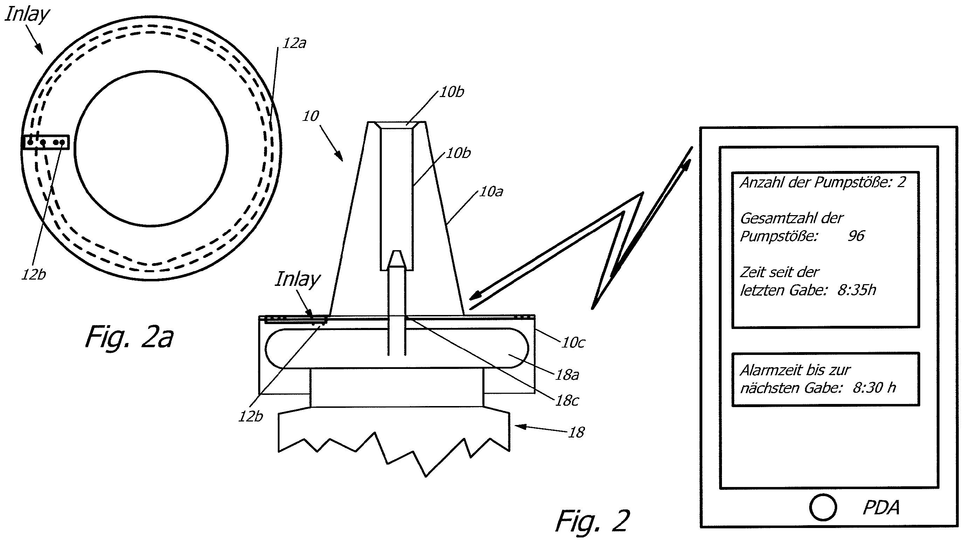

In den in Fig. 2 und 3 veranschaulichten Variante ist die Elektronikeinheit 12 nicht in das Kunststoffmaterial der Pumpkappe 10 eingebettet. Vielmehr ist hier die Elektronikeinheit 12 Teil eines separaten Einsatzes in Gestalt eines unabhängig von der Pumpkappe 10 herstellbaren Inlays für die Pumpkappe 10 für einen pharmazeutischen Behälter 18.

Der rohrförmige Abschnitt der Pumpkappe 10 ist an seinem einen (unteren) Ende offen und umgreift den Hals und ggf. teilweise den oberen Bereich des pharmazeutischen Behälters 18. Die Deckscheibe der kreiszylindrischen Aufnahme 10c verschließt den rohrförmigen Abschnitt ist an seinem anderen (oberen) Ende und wird zum Ausbringen des pharmazeutischen Mediums in Richtung auf die Metallkapsel 18a des pharmazeutischen Behälters 18 gepresst. An der Innenwand der Deckscheibe der kreiszylindrischen Aufnahme 10c befindet sich das zum Beispiel angeklebte oder durch Punktschweißung befestigte Inlay. Dieses auch in Fig. 2a veranschaulichte Inlay hat eine kreisringscheibenförmige Gestalt mit den Durchmesser-Abmessungen etwa einer 1-EURO-Münze mit einer hier runden, zentralen Durchtrittsöffnung (einige wenige Millimeter Durchmesser oder Querabmessung) für den Medikamenten-Austragsstutzen 18c des pharmazeutischen Behälters 18.

An seiner dem pharmazeutischen Behälter 18 zugewandten (in Fig. 2 der Unter-) Seite trägt das Inlay die Elektronikeinheit 12. Sie ist - vergleichbar mit der Variante aus Fig. 1 - mit einem Sensor 12b ausgestattet, der in einer Variante einen zur Erfassung eines Austragvorgangs eingerichteten Schalter hat. Dieser Schalter kann in der besonders einfachen Form durch zwei elektrische Kontaktstellen gebildet sein, die durch eine Metallkapsel 18a zum Verschluss des pharmazeutischen Behälters 18 dann elektrisch überbrückt werden, wenn der Benutzer die Pumpkappe 10 soweit herunterdrückt, dass ein Austragvorgang erfolgt. Sollte der Verschluss des pharmazeutischen Behälters 18 ohne Metallkapsel 18a, zum Beispiel in elektrisch nicht leitendem Kunststoff ausgeführt sein, kann der Sensor 12b ein als einfacher Öffner- oder Schließtaster ausgeführter Schalter ein. Anstelle dieses Schaltsensors kann auch ein kapazitiver, magnetischer oder sonstige Sensor 12b zur Erfassung des Pumpvorgangs vorgesehen sein, damit die Elektronikeinheit 12 ein den Medikamenten-Austragsvorgang entsprechend wiedergebendes Signal an den persönlichen digitalen Assistenten PDA abgeben kann.

Im Übrigen entspricht die Elektronikeinheit 12 der im Zusammenhang mit der Fig. 1 und 1a beschriebenen Variante. Das Folienmaterial des Einsatzes ist in der Variante der Fig. 2 eine Lage Kunststofffolie zum Beispiel aus Kunststoff, wie Polyethylen (PE) oder Polyvinylchlorid (PVC) mit einer Dicke von etwa 100 µm. Auf dieser Kunststofffolie ist die Antenne 12a, hier als Spulenantenne, aufgebracht und mit der Elektronikeinheit 12 kontaktiert, welche auch den Sensor 12b umfasst.

Bei der Variante von Fig. 3 ist im Unterschied zur Variante der Fig. 2 das Inlay mehrlagig ausgestaltet, wobei das Folienmaterial des Einsatzes in der Variante der Fig. 3 mehrere Lagen Kunststofffolie zum Beispiel aus Kunststoff, wie Polyethylen (PE) oder Polyvinylchlorid (PVC) mit einer Dicke von 75 µm aufeinander gestapelt hat. Zwischen den einzelnen Lagen befinden sich die Windungen der Spulenantenne 12a. In diesen Stapel aus Kunststofffolien ist in einer Variante neben der Antenne 12a auch der Betriebs-Energiespeicher 12c untergebracht. Zur Dimensionierung und Ausgestaltung der Spulenantenne 12a in ihren unterschiedlichen vorstehend dargestellten Varianten sei zum Beispiel auf "Antenna Circuit Design for RFID Applications", AN710, Youbok Lee, Ph.D., 2003 Microchip Technology Inc., verwiesen. Eine weitere Literaturstelle zur Dimensionierung und Ausgestaltung der der Spulenantenne 12a, auch abhängig von diversen unterschiedlichen in der Elektronikeinheit einsetzbaren RFID-Chips ist "RFID Design Fundamentals and Applications", Albert Lozano-Nieto, CRC Press Taylor & Francis Group 6000 Broken Sound Parkway NW, Suite 300 Boca Raton, FL 33487-2742, © 2011 by Taylor and Francis Group, LLC. Eine weitere Literaturstelle zur Dimensionierung und Ausgestaltung der Spulenantenne 12a ist "Design of Antennas for RFID Application", Ming-Tao Zhang et al. in "Development and Implementation of RFID Technology" edited by Cristina TURCU, ISBN 978-3-902613-54-7, pp. 554, February 2009, I-Tech, Wien, Österreich.

Die Pumpkappe mit der zugeordneten Elektronikeinheit 12 nach der Fig. 1 oder die Pumpkappe10 mit einem Inlay nach den Fig. 2 oder 3 ist mit einem pharmazeutischen Behälter 18 zu einem Gebinde zusammengefügt, und wobei sich in dem pharmazeutischen Behälter ein Nasenspray, beispielsweise in Form einer Lösung oder Suspension, bevorzugt Dymista® Nasenspray, eine Azelastinhydrochlorid und Fluticasonpropionat-Kombination, befindet, von der 1 g Suspension 1.000 Mikrogramm Azelastinhydrochlorid und 365 Mikrogramm Fluticasonpropionat enthält, und wobei ein Sprühstoß mit 0,14 g 137 Mikrogramm Azelastinhydrochlorid und 50 Mikrogramm Fluticasonpropionat enthält, und wobei die sonstigen Bestandteile des Nasensprays Dinatriumedetat, Glycerol, mikrokristalline Cellulose und Carmellose-Natrium, Polysorbat 80, Benzalkoniumchlorid, Phenylethylalkohol und gereinigtes Wasser sind.

Das in dem persönlichen digitalen Assistenten PDA ablaufende Computerprogrammprodukt bewirkt in einem Vorbereitungsmodus VBM die Paarung einer jeweiligen Elektronikeinheit 12 in (einem Inlay oder direkt in) der Pumpkappe 10 mit dem persönlichen digitalen Assistenten PDA. Dazu startet der Benutzer den Vorbereitungsmodus des Computerprogrammprodukts und bringt den persönlichen digitalen Assistenten PDA in räumliche Nähe zu der die Elektronikeinheit 12 enthaltende Pumpkappe 10. Im Vorbereitungsmodus VBM des Computerprogrammprodukts veranlasst dies die Abstrahlung von elektromagnetischer Energie nach dem NFC-Standard ISO / IEC 14443 A und B oder in ISO / IEC 15693 über die NFC-Funktionalität (Spulen, Ansteuerelektronik, entsprechender NFC-Ansteuersoftware (=NFC protocol stack) im Prozessor-Betriebssystem) des persönlichen digitalen Assistenten PDA. Sobald die Elektronikeinheit 12 über ihre Spulenantenne 12a ausreichende elektromagnetische Energie von der Antenne in dem persönlichen digitalen Assistenten PDA empfängt, wird die Elektronikeinheit 12 aktiviert und reagiert mit einem über ihre Spulenantenne 12a ausgesendeten Bestätigungssignal. Nun sendet der persönliche digitale Assistent PDA eine zum Beispiel (alphanumerische) mehrstellige individualisierte Kennung, die von der Elektronikeinheit 12 über die Antenne 12a empfangen und in einem hier nicht weiter veranschaulichten, nicht-flüchtigen EEPROM - Speicher des Chips der Elektronikeinheit 12 abgelegt wird.

Alternativ ist es auch möglich, dass nach dem Aktivieren der Elektronikeinheit 12 diese aus ihrem Speicher des Chips eine dort abgelegte zum Beispiel (alphanumerische) mehrstellige individualisierte Kennung über die Antenne 12a an den persönlichen digitalen Assistenten PDA aussendet.

In beiden Varianten wird erreicht, dass einerseits der persönliche digitale Assistent PDA und andererseits die Elektronikeinheit 12 in der Pumpkappe 10 aufeinander abgestimmt sind. So kann das Computerprogrammprodukt beim späteren Empfangen von Austragsvorgängen aus dem pharmazeutischen Behälter 18 durch Betätigen der Pumpkappe 10 wiedergebenden Signale diese Signale zuverlässig ausschließlich diesem, und keinem anderen Gebinde zuordnen und entsprechend verarbeiten. Außerdem wird ein Austragszähler auf null zurückgesetzt.

Um mit dem persönlichen digitalen Assistenten PDA und dem darauf ablaufenden Computerprogrammprodukt Austragsvorgänge aus dem pharmazeutischen Behälter 18 durch Betätigen der Pumpkappe 10 wiedergebende Signale zu erfassen, verarbeiten und auf dem Bildschirm des persönlichen digitalen Assistenten PDA anzeigen zu können, wird in einem Aufweckmodus AWM die Elektronikeinheit 12 in (einem Inlay oder direkt in) der Pumpkappe 10 aktiviert. Dazu startet der Benutzer das Computerprogrammprodukt und bringt den persönlichen digitalen Assistenten PDA in räumliche Nähe zu der die Elektronikeinheit 12 enthaltende Pumpkappe 10. Im Vorbereitungsmodus VBM des Computerprogrammprodukts veranlasst dies die Abstrahlung von elektromagnetischer Energie nach dem NFC-Standard ISO / IEC 14443 A und B oder in ISO / IEC 15693 über die NFC-Funktionalität (Spulen, Ansteuerelektronik, entsprechender NFC-Ansteuersoftware (=NFC protocol stack) im Prozessor-Betriebssystem) des persönlichen digitalen Assistenten PDA. Sobald die Elektronikeinheit 12 über ihre Spulenantenne 12a ausreichende elektromagnetische Energie von der Antenne in dem persönlichen digitalen Assistenten PDA empfängt, wird die Elektronikeinheit 12 aktiviert. Sobald die übertragene elektromagnetische Energie ausreicht, überträgt die Elektronikeinheit 12 ein kurzes "Aktivsignal" zusammen mit seiner individualisierten Kennung an den persönlichen digitalen Assistenten PDA. Dies erfolgt üblicherweise innerhalb einer oder zwei Sekunden, nachdem der Benutzer den persönlichen digitalen Assistenten PDA in räumliche Nähe zu der die Elektronikeinheit 12 enthaltende Pumpkappe 10 gebracht hat. Den Empfang des "Aktivsignals", und damit die Bereitschaft des persönlichen digitalen Assistenten PDA, Austragsvorgänge aus dem pharmazeutischen Behälter 18 durch Betätigen der Pumpkappe 10 wiedergebende Signale zu erfassen, verarbeiten und auf dem Bildschirm des persönlichen digitalen Assistenten PDA anzeigen zu können, zeigt das Computerprogrammprodukt durch einen entsprechenden akustischen oder visuellen Hinweis auf dem Bildschirm des persönlichen digitalen Assistenten PDA dar.

Im nächsten Schritt des Computerprogrammprodukts wird der persönliche digitale Assistent PDA im Empfangsmodus EM gehalten. In diesem Empfangsmodus EM wartet das Computerprogrammprodukt darauf, dass der Benutzer die Pumpkappe 10 betätigt, so dass die Elektronikeinheit 12 über ihre Spulenantenne 12a Signale übermittelt, die Austragsvorgänge aus dem pharmazeutischen Behälter 18 durch Betätigen der Pumpkappe 10 wiedergeben.

Damit die Zählung der Austragsvorgänge nicht von der Bedienung der Pumpkappe 10 durch den Benutzer abhängt, wird jede Betätigung der Pumpkappe 10 von der Elektronikeinheit 12 an den persönlichen digitalen Assistenten PDA übertragen. Das Computerprogrammprodukt wertet jedoch aus, wieviel Zeit zwischen zwei Betätigungen der Pumpkappe 10 vergeht. Sofern in sehr kurzen Abständen (etwa 1-4 Sekunden) zwei oder mehrere Betätigungen der Pumpkappe 10 von der Elektronikeinheit 12 signalisiert werden, wird dies von dem Computerprogrammprodukt als sog. Primen des pharmazeutischen Behälters 18 kategorisiert; das Computerprogrammprodukt erhöht den Austragszähler nicht, sondern zeigt den aktuellen Stand auf dem Bildschirm des persönlichen digitalen Assistenten PDA dar.

Werden zwei Betätigungen der Pumpkappe 10 von der Elektronikeinheit 12 in kurzem Abstand (etwa 4-10 Sekunden) an den persönlichen digitalen Assistenten PDA signalisiert, wird dies von dem Computerprogrammprodukt als zwei Austragsvorgänge (für jedes Nasenloch des Benutzers ein Austragsvorgang) kategorisiert; das Computerprogrammprodukt erhöht den Austragszähler entsprechend und zeigt den aktuellen Stand auf dem Bildschirm des persönlichen digitalen Assistenten PDA dar. Außerdem wird diese Anzahl der Austragsvorgänge zusammen mit einem aktuellen Datums-/Zeit-Stempel in dem Speicher des persönlichen digitalen Assistenten PDA zur späteren Verwendung abgespeichert.

Vorstehend ist eine Unterscheidung zwischen dem sog. Primen des pharmazeutischen Behälters 18 und zwei Austragsvorgängen durch einen unterschiedlichen zeitlichen Abstand zwischen zwei Betätigungen der Pumpkappe 10 beschrieben. In einer nicht weiter veranschaulichten Variante ist es vorgesehen, im Fall zweier Betätigungen der Pumpkappe 10, unabhängig von deren zeitlichem Abstand, in dem Computerprogrammprodukt den Austragszähler entsprechend zu erhöhen und den aktuellen Stand auf dem Bildschirm des persönlichen digitalen Assistenten PDA darzustellen. Werden mehr als zwei Betätigungen der Pumpkappe 10 innerhalb einer vorbestimmten Zeit, z.B. 5-15 Sekunden, an das Computerprogrammprodukt signalisiert, werden in einer weiteren, ebenfalls nicht detaillierter veranschaulichten Variante in dem Computerprogrammprodukt der Austragszähler um zwei erhöht und der aktuelle Stand auf dem Bildschirm des persönlichen digitalen Assistenten PDA dargestellt.

Eine weitere Variante sieht vor, nach der Paarung einer jeweiligen Elektronikeinheit 12 in (einem Inlay oder direkt in) der Pumpkappe 10 mit dem persönlichen digitalen Assistenten PDA in einem ersten Schritt mit einer Nachricht auf dem Bildschirm des persönlichen digitalen Assistenten PDA den Benutzer zum Primen aufzufordern. Dabei wird der signalisierte zeitliche Abstand erfasst und in dem Computerprogrammprodukt für ein Vergleichen des Signalverlaufs bei späteren Betätigungen der Pumpkappe 10 gespeichert. In einem zweiten Schritt wird mit einer Nachricht auf dem Bildschirm des persönlichen digitalen Assistenten PDA anschließend der Benutzer zum Ausführen zweier Austragsvorgänge (für jedes Nasenloch des Benutzers ein Austragsvorgang) aufgefordert. Dabei wird ebenfalls der signalisierte zeitliche Abstand erfasst und in dem Computerprogrammprodukt für ein Vergleichen des Signalverlaufs bei späteren Betätigungen der Pumpkappe 10 gespeichert.

Bei nachfolgenden Betätigungen der Pumpkappe 10 wird der dabei signalisierte zeitliche Verlauf der Betätigungen der Pumpkappe 10 mit den gespeicherten Verläufen verglichen. Je nach Kategorisierung der Betätigungen der Pumpkappe 10 wird dann in dem Computerprogrammprodukt der Austragszähler entsprechend um zwei erhöht (oder eben nicht, falls die Betätigungen als ein Primen kategorisiert wurden) und der aktuelle Stand auf dem Bildschirm des persönlichen digitalen Assistenten PDA dargestellt.

Zusätzlich zu den bisherig beschriebenen Funktionalitäten des Computerprogrammprodukts ist eine Historie-Darstellungsmodus HDM vorgesehen, der die seit der letzten Paarung einer jeweiligen Elektronikeinheit 12 der Pumpkappe 10 mit dem persönlichen digitalen Assistenten PDA gezählten Austragsvorgänge, jeweils mit einem Zeit-/Datumsstempel versehen auf dem Bildschirm des persönlichen digitalen Assistenten PDA darstellt. Dazu werden die in dem persönlichen digitalen Assistenten PDA abgespeicherten Austragsvorgänge zusammen mit ihrem jeweiligen Datums-/Zeit-Stempel aus dem Speicher des persönlichen digitalen Assistenten PDA ausgelesen und als Liste oder graphisch aufbereitet, zum Beispiel in einem Zeitstrahl angezeigt.

Des Weiteren ist ein Einnahme-Erinnerungsmodus EEM vorgesehen, in dem der Benutzer eine oder mehrere Tages-Uhrzeiten oder Zeitintervalle eingeben kann, zu denen der Benutzer jeweils die nächste Einnahme ausführen soll. Auf dem Bildschirm des persönlichen digitalen Assistenten PDA wird darstellt, wie viel Zeit bis zur nächsten Einnahme noch verbleibt. Zum vorgesehenen Zeitpunkt der Einnahme schlägt der persönliche digitale Assistent PDA optischen und/oder akustischen und/oder haptischen Alarm.

Die vorliegend beschriebenen Funktionalitäten des Computerprogrammprodukts können auch in ein Computerprogrammprodukt (App) eingebunden sein, das darüber hinaus noch eine Reihe weiterer Funktionen und Möglichkeiten für den Benutzer bereitstellt. So können beispielsweise verschiedene Symptome in ihrer Stärke und Häufigkeit dokumentiert und ggf. mit dem Verabreichungszyklus des Medikamentes korreliert werden.
Die vorangehend beschriebenen Varianten sowie deren Aufbau- und Betriebsaspekte dienen lediglich dem besseren Verständnis der Struktur, der Funktionsweise und der Eigenschaften; sie schränken die Offenbarung nicht etwa auf die Ausführungsbeispiele ein. Die Fig. sind teilweise schematisch, wobei wesentliche Eigenschaften und Effekte zum Teil deutlich vergrößert dargestellt sind, um die Funktionen, Wirkprinzipien, technischen Ausgestaltungen und Merkmale zu verdeutlichen. Dabei kann jede Funktionsweise, jedes Prinzip, jede technische Ausgestaltung und jedes Merkmal, welches/welche in den Fig. oder im Text offenbart ist / sind, mit allen Ansprüchen, jedem Merkmal im Text und in den anderen Fig., anderen Funktionsweisen, Prinzipien, technischen Ausgestaltungen und Merkmalen, die in dieser Offenbarung enthalten sind oder sich daraus ergeben, frei und beliebig kombiniert werden, so dass alle denkbaren Kombinationen der beschriebenen Varianten zuzuordnen sind. Dabei sind auch Kombinationen zwischen allen einzelnen Ausführungen im Text, das heißt in jedem Abschnitt der Beschreibung, in den Ansprüchen und auch Kombinationen zwischen verschiedenen Varianten im Text, in den Ansprüchen und in den Fig. umfasst. Auch die Ansprüche limitieren nicht die Offenbarung und damit die Kombinationsmöglichkeiten aller aufgezeigten Merkmale untereinander. Alle offenbarten Merkmale sind explizit auch einzeln und in Kombination mit allen anderen Merkmalen hier offenbart. Die Erfindung wird durch die folgenden Ansprüche definiert.

## Patentansprüche

1. Pumpkappeneinsatz für einen pharmazeutischen Behälter (18),
wobei dieser eine oder mehrere Kunststoff-Folien umfasst, deren Außendurchmesser geringer ist als der Innendurchmesser einer Pumpkappe (10), und die je eine Durchtrittsöffnung für einen Medikamenten-Austragsstutzen (18c) eines pharmazeutischen Behälters (18) haben, und
wobei der Einsatz eine Elektronikeinheit (12) aufweist, die ein Transmitter ist, der im Wesentlichen ein Chipmodul und eine als Spule oder als Dipol ausgestaltete Antenne (12a) hat, und
wobei den Kunststoff-Folien das Chipmodul des Transmitters und die als planare Ring-Spule oder als Dipol ausgestaltete Antenne (12a) zugeordnet ist, und
wobei die Elektronikeinheit (12) dazu eingerichtet ist,
- eine Betätigung der Pumpkappe (10) relativ zu dem pharmazeutischen Behälter (18) durch einen Benutzer zur Abgabe von Inhalt des pharmazeutischen Behälters (18) zu erfassen,
- Betätigungen der Pumpkappe (10) wiedergebende Signale drahtlos abzugeben, und
- Betriebs-Energie zum
(i) Erfassen der einen oder mehreren Betätigungen der Pumpkappe (10) durch einen Benutzer und zum
(ii) drahtlosen Abgeben von die eine oder mehrere Betätigungen der Pumpkappe (10) wiedergebenden Signale
sich von außen durch ein elektronisches Gerät in Gestalt eines persönlichen digitalen Assistenten (PDA) drahtlos zuführen zu lassen.

2. Pumpkappeneinsatz für einen pharmazeutischen Behälter (18) nach Anspruch 1 bei dem die Elektronikeinheit (12) in dem Einsatz zum berührungslosen, automatisierten Einschreiben und / oder Auslesen von Daten in den / aus dem Chip dient, und
wobei die einzuschreibenden Daten eine Personalisierungskennung des Einsatzes der Pumpkappe (10), zur Paarung mit einem Computerprogrammprodukt auf dem persönlichen digitalen Assistenten (PDA), und die auszulesenden Daten die Signale, welche die Betätigung der Pumpkappe (10) wiedergeben, umfassen.

3. Pumpkappeneinsatz für einen pharmazeutischen Behälter (18) nach einem der vorangegangen Ansprüche, bei der der Transmitter in den Einsatz integriert ist und dazu eingerichtet ist, seine Betriebsenergie von dem persönlichen digitalen Assistenten entweder nur zu empfangen, wenn der Benutzer das entsprechende Computerprogrammprodukt auf dem persönlichen digitalen Assistenten aktiviert, oder dazu eingerichtet ist, seine Betriebsenergie von dem persönlichen digitalen Assistenten (PDA) permanent zu empfangen, solange die Pumpkappe (10) mit dem Einsatz auf dem pharmazeutischen Behälter (18) sich in räumlicher Nähe zu dem persönlichen digitalen Assistenten befindet und dieser über seine entsprechenden Antenne(n) Energie abstrahlt.

4. Pumpkappeneinsatz für einen pharmazeutischen Behälter (18) nach einem der vorangegangenen Ansprüche, bei dem die Elektronikeinheit (12) dazu eingerichtet ist, die Betriebsenergie von dem persönlichen digitalen Assistenten in Gestalt eines Mobil- oder Schurlostelefons, eines sog. Smartphones, einer Smartwatch, eines Tabletcomputers, eines Notebooks, eines persönlichen digitalen Assistenten (PDA) oder dergl. zu empfangen, und/oder bei dem die Elektronikeinheit (12) einen Transmitter aufweist, der dazu eingerichtet ist, per Nahfeld-Kommunikation - NFC - Betätigungen der Pumpkappe (10) wiedergebende Signale drahtlos abzugeben, und /oder Betriebs-Energie drahtlos zu empfangen, und/oder bei dem die Elektronikeinheit (12) mit einem Sensor (12b) ausgestattet ist, der einen zur Erfassung eines Austragvorgangs eingerichteten Schalter, der vorzugsweise zwei elektrische Kontakte hat, die dazu eingerichtet und so angeordnet sind, dass sie durch einen metallischen Teil (18a) an dem pharmazeutischen Behälter (18) dann elektrisch überbrückt werden, wenn der Benutzer die Pumpkappe (10) mit dem darin befindlichen Einsatz soweit herunterdrückt, dass ein Austragvorgang erfolgt, oder der Sensor (12b) ein Öffner- oder Schließtaster ist, sofern der Sensor (12b) dazu eingerichtet ist, mit einem pharmazeutischen Behälter (18) zusammenzuwirken, dessen Verschluss ohne Metallkapsel (18a) ausgeführt ist.

5. Pumpkappeneinsatz für einen pharmazeutischen Behälter (18) nach einem der vorangegangenen Ansprüche, bei dem das Folienmaterial des Einsatzes eine oder mehrere Lagen holzfreies Papier, Beschichtungspapier oder mit Harz imprägniertes Papier, Kunststoff- bzw. Plastikfolie aus Kunststoff, wie Polyethylen (PE), Polyvinylchlorid (PVC), Polyvinylchlorid-Acetat-Copolymer, Polyethylenterephthalat (PET) oder Glykol-modifiziertes Polyethylenterephthalat (PETG), Polyethylennaphthalat (PEN), Acrylnitril-Butadien-Styrol-Copolymerisat (ABS), Polyvinylbutyral (PVB), Polymethyl-methacrylat (PMMA), Polyimid (PI), Polyvinylalkohol (PVA), Polystyrol (PS), Polyvinylphenol (PVP), Polyethylen (PE), Polypropylen (PP), Polycarbonat (PC) oder deren Derivate enthaltende Folien mit einer Dicke von mind. 75µm oder mehr ist.

6. Pumpkappe (10) für einen pharmazeutischen Behälter (18), umfassend einen Pumpkappeneinsatz nach Anspruch 1.

7. Pumpkappe (10) für einen pharmazeutischen Behälter (18) nach Anspruch 6,
bei dem die Elektronikeinheit (12) in dem Einsatz zum berührungslosen, automatisierten Einschreiben und / oder Auslesen von Daten in den / aus dem Chip dient, und
wobei die einzuschreibenden Daten eine Personalisierungskennung zur Paarung der Elektronikeinheit (12) mit einem Computerprogrammprodukt auf dem persönlichen digitalen Assistenten (PDA), und /oder die auszulesenden Daten die Signale, welche die Betätigung der Pumpkappe (10) wiedergeben, umfassen.

8. Pumpkappe (10) für einen pharmazeutischen Behälter (18) nach Anspruch 7, bei der der Transmitter dazu eingerichtet ist, seine Betriebsenergie von dem persönlichen digitalen Assistenten entweder nur zu empfangen, wenn der Benutzer das entsprechende Computerprogrammprodukt auf dem persönlichen digitalen Assistenten aktiviert, oder dazu eingerichtet ist, seine Betriebsenergie von dem persönlichen digitalen Assistenten permanent zu empfangen, solange die Pumpkappe (10) auf dem pharmazeutischen Behälter (18) sich in räumlicher Nähe zu dem persönlichen digitalen Assistenten befindet und dieser über seine entsprechenden Antenne(n) Energie abstrahlt.

9. Pumpkappe (10) für einen pharmazeutischen Behälter (18) nach den Ansprüchen 6 bis 8 bei der die Elektronikeinheit (12) dazu eingerichtet ist, die Betriebsenergie von dem persönlichen digitalen Assistenten, eines sog. persönlichen digitalen Assistenten (PDA), in Gestalt eines Mobil- oder Schurlostelefons, eines sog. Smartphones, einer Smartwatch, eines Tablet-computers, eines Notebooks, oder dergl. zu empfangen, und/oder bei der die Elektronikeinheit (12) einen Transmitter aufweist, der dazu eingerichtet ist, per Nahfeld-Kommunikation - NFC - Betätigungen der Pumpkappe (10) wiedergebende Signale drahtlos abzugeben, und /oder Betriebs-Energie drahtlos zu empfangen, und/oder bei der die Elektronikeinheit (12) im Pumpkappeneinsatz mit einem Sensor (12b) ausgestattet ist, der einen zur Erfassung eines Austragvorgangs eingerichteten Schalter, der vorzugsweise wenigstens zwei elektrische Kontakte hat, die dazu eingerichtet und so angeordnet sind, dass sie durch einen metallischen Teil (18a) an dem pharmazeutischen Behälter (18) oder der Pumpkappe (10) dann elektrisch überbrückt werden, wenn der Benutzer die Pumpkappe (10) soweit herunterdrückt, dass ein Austragvorgang erfolgt, oder der Schalter ein Öffner- oder Schließtaster ist, sofern der Sensor (12b) dazu eingerichtet ist, mit dem pharmazeutischen Behälter zusammenzuwirken, dessen Verschluss ohne Metallkapsel (18a) ausgeführt ist.

10. Pharmazeutischer Behälter (18) mit einem Pumpkappeneinsatz nach einem der Ansprüche 1 - 5 oder einer Pumpkappe (10) nach einem der Ansprüche 6 bis 9.

11. Pharmazeutischer Behälter (18) nach Anspruch 10, wobei sich in dem pharmazeutischen Behälter ein Nasenspray, beispielsweise in Form einer Lösung oder Suspension, bevorzugt Dymista® Nasenspray, eine AzelastinHCl/Fluticasonpropionat-Kombination, befindet.

12. Computerprogrammprodukt, welches durch entsprechende Ansteuerung einer Antennen-Ansteuerelektronik eines persönlichen digitalen Assistenten (PDA) veranlasst, eine Elektronikeinheit (12) in einem Inlay oder direkt in einer Pumpkappe (10) nach einem der Ansprüche 6-9 mit Betriebsenergie zu versorgen und Austragvorgänge aus dem pharmazeutischen Behälter (18) nach einem der Ansprüche 10-11 zu erfassen, mit
- einem Vorbereitungsmodus (VBM), um eine Paarung einer jeweiligen Elektronikeinheit (12) in einem Inlay bzw. in der Pumpkappe (10) mit dem persönlichen digitalen Assistenten zu bewirken,
- einem Aufweckmodus (AWM), um die Elektronikeinheit (12) in dem Inlay bzw. in der Pumpkappe (10) zu aktivieren,
- einem Empfangsmodus (EM), um darauf zu warten, dass der Benutzer die Pumpkappe (10) betätigt, so dass die Elektronikeinheit (12) Signale übermittelt, die Austragsvorgänge aus dem pharmazeutischen Behälter (18) durch Betätigen der Pumpkappe (10) wiedergeben,
- einem Auswertemodus (AM), um verstrichene Zeit zwischen aufeinanderfolgenden Betätigungen der Pumpkappe (10) zu messen und abhängig von der verstrichenen Zeit unterschiedliche Ereignisse zu kategorisieren.

13. Computerprogrammprodukt nach Anspruch 12, bei dem in dem Vorbereitungsmodus (VBM)
- die Abstrahlung von elektromagnetischer Energie durch den persönlichen digitalen Assistenten (PDA) bewirkt wird, damit die Elektronikeinheit (12) über ihre Spulenantenne (12a) elektromagnetische Energie von der Antenne in dem persönlichen digitalen Assistenten (PDA) empfängt, worauf die Elektronikeinheit (12) aktiviert wird und mit einem über ihre Spulenantenne (12a) ausgesendeten Bestätigungssignal reagiert;
- eine individualisierte Kennung ausgesendet wird, damit diese von der Elektronikeinheit (12) über deren Antenne empfangen und in der Elektronikeinheit (12) abgelegt wird, oder die Elektronikeinheit (12) eine darin abgelegte individualisierte Kennung über ihre Antenne an den persönlichen digitalen Assistenten (PDA) aussendet, und
- ein Austragszähler auf null gesetzt wird.

14. Computerprogrammprodukt nach Anspruch 12 oder 13, bei dem
in dem Aufweckmodus (AWM)
- die Abstrahlung von elektromagnetischer Energie durch den persönlichen digitalen Assistenten (PDA) bewirkt wird, damit die Elektronikeinheit (12) über ihre Spulenantenne (12a) elektromagnetische Energie von der Antenne in dem persönlichen digitalen Assistenten (PDA) empfängt und ein "Aktivsignal" zusammen mit seiner individualisierten Kennung an den persönlichen digitalen Assistenten (PDA) überträgt; und
- nach dem Empfang des "Aktivsignals" ein Audio-und/oder visueller Hinweis durch den persönlichen digitalen Assistenten (PDA) ausgegeben wird, um die Bereitschaft des persönlichen digitalen Assistenten (PDA) anzugeben, Austragsvorgänge aus dem pharmazeutischen Behälter durch Betätigen der Pumpkappe (10) wiedergebende Signale zu erfassen, verarbeiten und/oder auf dem Bildschirm des persönlichen digitalen Assistenten (PDA) anzuzeigen, und/oder
in dem Auswertemodus (AM)
- bei in sehr kurzem zeitlichem Abstand von zwei oder mehreren von der Elektronikeinheit (12) signalisierten Betätigungen der Pumpkappe (10), dies als Primen des pharmazeutischen Behälters (18) kategorisiert wird, und der Austragszähler nicht erhöht wird;
- bei in kurzem zeitlichem Abstand zweier oder mehrerer von der Elektronikeinheit (12) signalisierten Betätigungen der Pumpkappe (10), dies als zwei Austragsvorgänge kategorisiert wird, und der Austragszähler entsprechend erhöht wird; und
- diese Anzahl der Austragsvorgänge zusammen mit einem aktuellen Datums-/Zeit-Stempel in dem persönlichen digitalen Assistenten PDA abgespeichert wird, und/oder
- in einem Historie-Darstellungsmodus (HDM) die seit der letzten Paarung einer jeweiligen Elektronikeinheit (12) der Pumpkappe mit dem persönlichen digitalen Assistenten (PDA) gezählten Austragsvorgänge, jeweils mit einem Zeit-/Datumsstempel versehen, auf dem Bildschirm des persönlichen digitalen Assistenten (PDA) dargestellt werden, und / oder
- in einem Einnahme-Erinnerungsmodus (EEM) der Benutzer in den persönlichen digitalen Assistenten (PDA) eine oder mehrere Tages-Uhrzeiten oder Zeitintervalle eingeben kann, zu denen der Benutzer jeweils die nächste Einnahme ausführen soll, wobei
- auf dem Bildschirm des persönlichen digitalen Assistenten PDA darstellt wird, wie viel Zeit bis zur nächsten Einnahme noch verbleibt, und/oder
- zum vorgesehenen Zeitpunkt der Einnahme der persönliche digitale Assistent PDA optischen und/oder akustischen und/oder haptischen Alarm schlägt.

## Claims

1. Pump cap insert for a pharmaceutical container (18),
wherein said pump cap insert comprises one or more plastic films, the outer diameter of which is smaller than the inner diameter of a pump cap (10) and which each have a through-opening for a medicine discharge nozzle (18c) of a pharmaceutical container (18), and
wherein the insert comprises an electronics unit (12), which is a transmitter that essentially has a chip module and an antenna (12a) designed as a coil or as a dipole, and
wherein the chip module of the transmitter and the antenna (12a) designed as a planar ring coil or as a dipole is assigned to the plastic films, and
wherein the electronics unit (12) is configured to
- detect an actuation of the pump cap (10) relative to the pharmaceutical container (18) by a user for dispensing content of the pharmaceutical container (18),
- wirelessly emit signals representing actuations of the pump cap (10), and
- have operating energy for
(i) detecting the one or more actuations of the pump cap (10) by a user and for
(ii) wirelessly emitting signals representing the one or more actuations of the pump cap (10)
wirelessly supplied to itself from the outside by an electronic device in the form of a personal digital assistant (PDA).

2. Pump cap insert for a pharmaceutical container (18) according to claim 1, wherein the electronics unit (12) in the insert is used for the contactless, automated writing and/or reading of data into/out of the chip, and
wherein the data to be written includes a personalisation identifier for the insert of the pump cap (10) for pairing with a computer program product on the personal digital assistant (PDA), and the data to be read includes the signals which represent the actuation of the pump cap (10).

3. Pump cap insert for a pharmaceutical container (18) according to any one of the preceding claims, in which the transmitter is integrated into the insert and is configured to either only receive its operating energy from the personal digital assistant when the user activates the relevant computer program product on the personal digital assistant, or is configured to permanently receive its operating energy from the personal digital assistant (PDA) as long as the pump cap (10) with the insert on the pharmaceutical container (18) is in spatial proximity to the personal digital assistant and said personal digital assistant radiates energy via its corresponding antenna(s).

4. Pump cap insert for a pharmaceutical container (18) according to any one of the preceding claims, in which the electronics unit (12) is configured to receive the operating energy from the personal digital assistant in the form of a mobile or cordless telephone, a so-called smartphone, a smart watch, a tablet computer, a notebook, a personal digital assistant (PDA) or the like, and/or in which the electronics unit (12) comprises a transmitter which is configured to wirelessly emit signals representing actuations of the pump cap (10) via near-field communication (NFC), and/or wirelessly receive operating energy, and/or in which the electronics unit (12) is equipped with a sensor (12b) having a switch that is configured to detect a discharge operation and preferably has two electrical contacts which are configured and arranged such that they are electrically bypassed via a metallic part (18a) on the pharmaceutical container (18) when the user presses the pump cap (10) with the insert located therein to such an extent that a discharge operation takes place, or the sensor (12b) is an opening or closing button if the sensor (12b) is configured to interact with a pharmaceutical container (18), the closure of which is constructed without a metal capsule (18a).

5. Pump cap insert for a pharmaceutical container (18) according to any one of the preceding claims, in which the film material of the insert is one or more layers of wood-free paper, coating paper or paper impregnated with resin, plastic film made of plastic, such as polyethylene (PE), polyvinyl chloride (PVC), polyvinyl chloride acetate copolymer, polyethylene terephthalate (PET) or glycol-modified polyethylene terephthalate (PETG), polyethylene naphthalate (PEN), acrylonitrile butadiene styrene copolymerisate (ABS), polyvinyl butyral (PVB), polymethyl methacrylate (PMMA), polyimide (PI), polyvinyl alcohol (PVA), polystyrene (PS), polyvinyl phenol (PVP), polyethylene (PE), polypropylene (PP), polycarbonate (PC) or films containing derivatives thereof having a thickness of at least 75 µm or more.

6. Pump cap (10) for a pharmaceutical container (18), comprising a pump cap insert according to claim 1.

7. Pump cap (10) for a pharmaceutical container (18) according to claim 6,
in which the electronics unit (12) in the insert is used for the contactless, automated writing and/or reading of data into/out of the chip, and
wherein the data to be written includes a personalisation identifier for pairing the electronics unit (12) with a computer program product on the personal digital assistant (PDA), and/or the data to be read includes the signals which represent the actuation of the pump cap (10).

8. Pump cap (10) for a pharmaceutical container (18) according to claim 7, in which the transmitter is configured to either only receive its operating energy from the personal digital assistant when the user activates the relevant computer program product on the personal digital assistant, or is configured to permanently receive its operating energy from the personal digital assistant as long as the pump cap (10) on the pharmaceutical container (18) is in spatial proximity to the personal digital assistant and said personal digital assistant radiates energy via its corresponding antenna(s).

9. Pump cap (10) for a pharmaceutical container (18) according to claims 6 to 8, in which the electronics unit (12) is configured to receive the operating energy from the personal digital assistant, a so-called personal digital assistant (PDA), in the form of a mobile or cordless telephone, a so-called smartphone, a smart watch, a tablet computer, a notebook, or the like, and/or in which the electronics unit (12) comprises a transmitter which is configured to wirelessly emit signals representing actuations of the pump cap (10) via near-field communication (NFC), and/or wirelessly receive operating energy, and/or in which the electronics unit (12) in the pump cap insert is equipped with a sensor (12b) having a switch that is configured to detect a discharge operation and preferably has at least two electrical contacts which are configured and arranged such that they are electrically bypassed via a metallic part (18a) on the pharmaceutical container (18) or the pump cap (10) when the user presses the pump cap (10) to such an extent that a discharge operation takes place, or the switch is an opening or closing button if the sensor (12b) is configured to interact with the pharmaceutical container, the closure of which is constructed without a metal capsule (18a).

10. Pharmaceutical container (18) having a pump cap insert according to any one of claims 1 - 5 or a pump cap (10) according to any one of claims 6 to 9.

11. Pharmaceutical container (18) according to claim 10, wherein the pharmaceutical container contains a nasal spray, for example in the form of a solution or suspension, preferably Dymista® nasal spray, an azelastine HCl/fluticasone propionate combination.

12. Computer program product which, via the corresponding control of an antenna electronic control system of a personal digital assistant (PDA), causes an electronics unit (12) in an inlay or directly in a pump cap (10) according to any one of Claims 6-9 to be supplied with operating energy and detect discharge operations from the pharmaceutical container (18) according to any one of claims 10-11, having
a preparation mode (VBM), to achieve a pairing of a respective electronics unit (12) in an inlay or in the pump cap (10) with the personal digital
assistant,
- a wake-up mode (AWM), to activate the electronics unit (12) in the inlay or the pump cap (10),
- a receiving mode (EM), to wait for the user to actuate the pump cap (10), so that the electronics unit (12) transmits signals that represent discharge operations from the pharmaceutical container (18) via the actuation of the pump cap (10),
- an evaluation mode (AM), to measure the time elapsed between successive actuations of the pump cap (10) and to categorise different events as a function of the elapsed time.

13. Computer program product according to claim 12, in which, in the preparation mode (VBM)
- the radiation of electromagnetic energy by the personal digital assistant (PDA) is effected, so that the electronics unit (12) receives electromagnetic energy from the antenna in the personal digital assistant (PDA) via its coil antenna (12a), whereupon the electronics unit (12) is activated and reacts with an acknowledgement signal transmitted via its coil antenna (12a);
- an individualised identifier is transmitted, so that said identifier is received by the electronics unit (12) via its antenna and stored in the electronics unit (12), or the electronics unit (12) transmits an individualised identifier stored in said unit to the personal digital assistant (PDA) via its antenna, and
- a discharge counter is set to zero.

14. Computer program product according to claim 12 or 13, in which,
in the wake-up mode (AWM)
- the radiation of electromagnetic energy by the personal digital assistant (PDA) is effected, so that the electronics unit (12) receives electromagnetic energy from the antenna in the personal digital assistant (PDA) via its coil antenna (12a) and transmits an "active signal" to the personal digital assistant (PDA) along with its individualised identifier; and
- after receiving the "active signal", an audio and/or visual notification is emitted by the personal digital assistant (PDA) to indicate the readiness of the personal digital assistant (PDA) to acquire signals that represent discharge operations from the pharmaceutical container via the actuation of the pump cap (10), process said signals and/or display them on the screen of the personal digital assistant (PDA),
and/or,
in the evaluation mode (AM)
- in the event of a very short time interval between two or more actuations of the pump cap (10) signalled by the electronics unit (12), said actuations are categorised as priming of the pharmaceutical container (18), and the discharge counter is not advanced;
- in the event of a very short time interval between two or more actuations of the pump cap (10) signalled by the electronics unit (12), said actuations are categorised as two discharge operations, and the discharge counter is advanced accordingly; and
- said number of discharge operations is stored in the personal digital assistant PDA along with a current date/time stamp, and/or
- in a history display mode (HDM), the discharge operations counted since the last pairing of a respective electronics unit (12) of the pump cap with the personal digital assistant (PDA), and in each case provided with a time/date stamp, are displayed on the screen of the personal digital assistant (PDA), and/or,
- in an administration reminder mode (EEM), the user can enter one or more times of day or time intervals at which said user is supposed to take the next respective dose into the personal digital assistant (PDA), wherein
- the time remaining until the next dose is displayed on the screen of the personal digital assistant PDA, and/or
- the personal digital assistant PDA provides a visual and/or acoustic and/or haptic alert at the scheduled time for the dose.

## Revendications

1. Insert de capuchon de pompage pour un récipient pharmaceutique (18),
comprenant une ou plusieurs feuilles de matière synthétique dont le diamètre externe est inférieur au diamètre interne d'un capuchon de pompage (10) et qui présentent chacune une ouverture traversante pour un raccord de distribution de médicament (18c) d'un récipient pharmaceutique (18),
l'insert présentant une unité électronique (12) consistant en un émetteur et possédant essentiellement un module de puce et une antenne (12a) sous la forme d'une bobine ou d'un dipôle, et le module de puce de l'émetteur et l'antenne (12a) conçue sous la forme d'une bobine annulaire plane ou d'un dipôle étant attribués aux feuilles de matière synthétique, et
l'unité électronique (12) étant conçue pour
- déterminer un actionnement du capuchon de pompage (10) par rapport au récipient pharmaceutique (18) par un utilisateur pour la distribution du contenu du récipient pharmaceutique (18),
- émettre sans fil des signaux indicateurs des actionnements du capuchon de pompage (10), et
- recevoir de l'énergie de fonctionnement depuis l'extérieur par le biais d'un appareil électronique sous la forme d'un assistant numérique personnel (PDA) pour
(i) déterminer le ou les actionnements du capuchon de pompage (10) par un utilisateur et pour
(ii) émettre sans fil des signaux indicateurs du ou des actionnements du capuchon de pompage (10).

2. Insert de capuchon de pompage pour un récipient pharmaceutique (18) selon la revendication 1, dans lequel l'unité électronique (12) de l'insert sert à l'écriture et/ou la lecture automatisées sans contact de données sur la puce, et
dans lequel les données à écrire comprennent un identifiant de personnalisation de l'insert du capuchon de pompage (10), pour l'appariement avec un produit de programme informatique présent sur l'assistant numérique personnel (PDA), et les données à lire comprennent les signaux indicateurs de l'actionnement du capuchon de pompage (10).

3. Insert de capuchon de pompage pour un récipient pharmaceutique (18) selon une des revendications précédentes, dans lequel l'émetteur est intégré dans l'insert et est conçu pour recevoir son énergie de fonctionnement de l'assistant numérique personnel uniquement lorsque l'utilisateur active le produit de programme informatique correspondant sur l'assistant numérique personnel, ou est conçu pour recevoir en permanence son énergie de fonctionnement de l'assistant numérique personnel (PDA), tant que le capuchon de pompage (10) présentant l'insert sur le récipient pharmaceutique (18) se trouve à proximité spatiale de l'assistant numérique personnel et que celui-ci rayonne de l'énergie par le biais de sa ou de ses antennes correspondantes.

4. Insert de capuchon de pompage pour un récipient pharmaceutique (18) selon une des revendications précédentes, dans lequel l'unité électronique (12) est conçue pour recevoir l'énergie de fonctionnement de l'assistant numérique personnel consistant en un téléphone mobile ou sans fil, un smartphone, une montre connectée, un ordinateur tablette, un ordinateur portable, un assistant numérique personnel (PDA) ou analogue et/ou dans lequel l'unité électronique (12) présente un émetteur conçu pour émettre sans fil des signaux indicateurs des actionnements du capuchon de pompage (10) par communication en champ proche - NFC - et/ou pour recevoir son énergie de fonctionnement sans fil, et/ou dans lequel l'unité électronique (12) est équipée d'un capteur (12b) qui possède un commutateur qui est destiné à déterminer un processus de distribution et qui possède de préférence deux contacts électriques conçus et disposés de manière à être reliés électriquement par une partie métallique (18a) au récipient pharmaceutique (18) lorsque l'utilisateur enfonce le capuchon de pompage (10) renfermant de l'insert jusqu'à ce qu'un processus de distribution se produise, ou bien le capteur (12b) consiste en un capteur d'ouverture ou de fermeture, dans la mesure où le capteur (12b) est conçu pour coopérer avec un récipient pharmaceutique (18) dont la fermeture s'effectue sans capsule métallique (18a).

5. Insert de capuchon de pompage pour un récipient pharmaceutique (18) selon une des revendications précédentes, dans lequel le matériau en feuille de l'insert consiste en une ou plusieurs couches de papier sans bois, de papier de revêtement ou de papier imprégné de résine, des feuilles de matières synthétiques ou plastiques telles que le polyéthylène (PE), le polychlorure de vinyle (PVC), le copolymère de polychlorure de vinyle-acétate, le poly(téréphtalate d'éthylène) (PET) ou le poly(téréphtalate d'éthylène) modifié par un glycol (PETG), le polynaphtalate d'éthylène (PEN), le copolymère d'acrylonitrile-butadiène-styrène (ABS), le poly(butyral vinylique) (PVB), le polyméthacrylate de méthyle (PMMA), le poly-imide (PI), l'alcool polyvinylique (PVA), le polystyrène (PS), le polyvinylphénol (PVP), le polyéthylène (PE), le polypropylène (PP), le polycarbonate (PC) ou consiste en des feuilles contenant des dérivés de ces matériaux, avec une épaisseur d'au moins 75 µm ou plus.

6. Capuchon de pompage (10) pour un récipient pharmaceutique (18) comprenant un insert de capuchon de pompage selon la revendication 1.

7. Capuchon de pompage (10) pour un récipient pharmaceutique (18) selon la revendication 6,
dans lequel l'unité électronique (12) de l'insert sert à l'écriture et/ou la lecture automatisées sans contact de données sur la puce, et
dans lequel les données à écrire comprennent un identifiant de personnalisation pour l'appariement de l'unité électronique (12) avec un produit de programme informatique présent sur l'assistant numérique personnel (PDA), et/ou les données à lire comprennent les signaux indicateurs de l'actionnement du capuchon de pompage (10).

8. Capuchon de pompage (10) pour un récipient pharmaceutique (18) selon la revendication 7, dans lequel l'émetteur est conçu pour recevoir son énergie de fonctionnement de l'assistant numérique personnel uniquement lorsque l'utilisateur active le produit de programme informatique correspondant sur l'assistant numérique personnel, ou est conçu pour recevoir en permanence son énergie de fonctionnement de l'assistant numérique personnel tant que le capuchon de pompage (10) présent sur le récipient pharmaceutique (18) se trouve à proximité spatiale de l'assistant numérique personnel et que celui-ci rayonne de l'énergie par le biais de sa ou de ses antennes correspondantes.

9. Capuchon de pompage (10) pour un récipient pharmaceutique (18) selon les revendications 6 à 8, dans lequel l'unité électronique (12) est conçue pour recevoir l'énergie de fonctionnement de l'assistant numérique personnel, d'un assistant numérique personnel (PDA) consistant en un téléphone mobile ou sans fil, un smartphone, une montre connectée, un ordinateur tablette, un ordinateur portable ou analogue, et/ou dans lequel l'unité électronique (12) présente un émetteur qui est conçu pour émettre sans fil des signaux indicateurs des actionnements du capuchon de pompage (10) par communication en champ proche - NFC - et/ou pour recevoir l'énergie de fonctionnement sans fil, et/ou dans lequel l'unité électronique (12) de l'insert de capuchon de pompage est équipée d'un capteur (12b), qui possède un commutateur qui est destiné à déterminer un processus de distribution et qui possède de préférence au moins deux contacts électriques conçus et disposés de manière à être reliés électriquement par une partie métallique (18a) au récipient pharmaceutique (18) ou au capuchon de pompage (10) lorsque l'utilisateur enfonce le capuchon de pompage (10) jusqu'à ce qu'un processus de distribution se produise, ou le commutateur est un interrupteur d'ouverture ou de fermeture, dans la mesure où le capteur (12b) est conçu pour coopérer avec le récipient pharmaceutique dont la fermeture s'effectue sans capsule métallique (18a).

10. Récipient pharmaceutique (18) comprenant un insert de capuchon de pompage selon une des revendications 1 à 5 ou un capuchon de pompage (10) selon une des revendications 6 à 9.

11. Récipient pharmaceutique (18) selon la revendication 10, renfermant un spray nasal, par exemple sous la forme d'une solution ou d'une suspension, de préférence le spray nasal Dymista®, une combinaison de chlorhydrate d'azélastine/propionate de fluticasone.

12. Produit de programme informatique qui, par une commande correspondante d'une électronique de commande d'antennes d'un assistant numérique personnel (PDA), provoque l'apport d'énergie de fonctionnement auprès d'une unité électronique (12) présente dans un insert ou directement dans un capuchon de pompage (10) selon une des revendications 6 à 9, et la détermination de processus de distribution réalisés par le récipient pharmaceutique (18) selon une des revendications 10 et 11, lequel produit de programme informatique comprend :
- un mode de préparation (MP) destiné à réaliser un appariement d'une unité électronique (12) respective d'un insert ou du capuchon de pompage (10) avec l'assistant numérique personnel,
- un mode de réveil (MRV) destiné à activer l'unité électronique (12) de l'insert ou du capuchon de pompage (10),
- un mode de réception (MRC) destiné à attendre que l'utilisateur actionne le capuchon de pompage (10) de sorte que l'unité électronique (12) transmette des signaux indicateurs des processus de distribution réalisés par le récipient pharmaceutique (18) grâce à l'actionnement du capuchon de pompage (10),
- un mode d'évaluation (ME) destiné à mesurer le temps écoulé entre deux actionnements successifs du capuchon de pompage (10) et à catégoriser différents résultats en fonction du temps écoulé.

13. Produit de programme informatique selon la revendication 12, dans lequel, dans le mode de préparation (MP),
- le rayonnement d'énergie électromagnétique est provoqué par l'assistant numérique personnel (PDA) afin que l'unité électronique (12) reçoive de l'énergie électromagnétique par le biais de son antenne en bobine (12a) en provenance de l'antenne de l'assistant numérique personnel (PDA), moyennant quoi l'unité électronique (12) est activée et réagit à un signal de confirmation envoyé par le biais de son antenne en bobine (12a),
- un identifiant individualisé est envoyé afin d'être reçu par l'unité électronique (12) par le biais de ses antennes puis stocké dans l'unité électronique (12), ou bien l'unité électronique (12) envoie un identifiant individualisé stocké, par le biais de son antenne auprès de l'assistant numérique personnel (PDA), et
- un compteur de distribution est mis à zéro.

14. Produit de programme informatique selon la revendication 12 ou 13, dans lequel,
dans le mode de réveil (MRV),
- le rayonnement d'énergie électromagnétique est provoqué par l'assistant numérique personnel (PDA) afin que l'unité électronique (12) reçoive de l'énergie électromagnétique par le biais de son antenne en bobine (12a) en provenance de l'antenne de l'assistant numérique personnel (PDA) et transmette un « signal d'activation » conjointement avec son identifiant individualisé auprès de l'assistant numérique personnel (PDA), et
- après la réception du « signal d'activation », un avertissement sonore et/ou visuel est émis par l'assistant numérique personnel (PDA) pour annoncer la disponibilité de l'assistant numérique personnel (PDA) à déterminer ou traiter les signaux indicateurs de processus de distribution réalisés par le récipient pharmaceutique grâce à l'actionnement du capuchon de pompage (10) et/ou à les afficher sur l'écran de l'assistant numérique personnel (PDA),
et/ou
dans le mode d'évaluation (ME),
- en cas de signalisation par l'unité électronique (12) d'au moins deux actionnements du capuchon de pompage (10) à très faible intervalle, cela est catégorisé comme une amorce du récipient pharmaceutique (18) et le compteur de distribution n'augmente pas,
- en cas de signalisation par l'unité électronique (12) d'au moins deux actionnements du capuchon de pompage (10) à faible intervalle, cela est catégorisé comme deux processus de distribution et le compteur de distribution augmente en conséquence ; et
- ce nombre de processus de distribution est horodaté et mémorisé dans l'assistant numérique personnel PDA, et/ou
- dans un mode de représentation d'historique (MRH), les processus de distribution respectivement horodatés et comptabilisés depuis le dernier appariement d'une unité électronique (12) respective du capuchon de pompage avec l'assistant numérique personnel (PDA) sont affichés sur l'écran de l'assistant numérique personnel (PDA), et/ou
- dans un mode de rappel d'administration (MRA), l'utilisateur peut saisir dans l'assistant numérique personnel (PDA) une ou plusieurs heures de la journée ou un ou plusieurs intervalles temporels auxquels il doit effectuer la prochaine administration, en sachant que :
- le temps restant jusqu'à la prochaine administration est affiché sur l'écran de l'assistant numérique personnel PDA, et/ou
- à l'horaire prévu de l'administration, l'assistant numérique personnel PDA émet une alarme optique et/ou acoustique et/ou haptique.
